# EUROPEAN PATENT SPECIFICATION

(11) **EP 3 185 893 B1**
(45) Date of publication and mention of the grant of the patent: **05.07.2023**
(21) Application number: 15739689.6
(22) Date of filing: 12.06.2015
(51) Int. Cl.: A61P 31/16, A61K 39/12, A61K 39/145

(54) **USE OF CATIONIC DERIVATIVES OF POLYPRENOLS PTAI IN PRODUCTION OF IMMUNOMODULATING SUBSTANCES**
VERWENDUNG VON KATIONISCHEN DERIVATEN VON POLYPRENOL-PTAI BEI DER HERSTELLUNG VON IMMUNMODULIERENDEN STOFFEN
UTILISATION DE DÉRIVÉS CATIONIQUES DE POLYPRÉNOLS PTAI DANS LA PRODUCTION DE SUBSTANCES IMMUNOMODULATRICES

(30) Priority: 28.08.2014 PL 40929814; 04.11.2014 PL 41006314
(43) Date of publication of application: 05.07.2017
(73) Proprietor: Instytut Biochemii i Biofizyki PAN, 02-106 Warszawa (PL); Uniwersytet Jagiellonski, 31-007 Krakow (PL); Instytut Chemii Organicznej PAN, 01-224 Warszawa (PL)
(72) Inventor: GÓRA-SOCHACKA, Anna, PL-02-495 Warszawa (PL); STACHYRA, Anna, PL-04-998 Warszawa (PL); SIRKO, Agnieszka, PL-01-227 Warszawa (PL); ZAGÓRSKI-OSTOJA, Wlodzimierz, PL-02-665 Warszawa (PL); REDKIEWICZ, Patrycja, PL-05-240 Tluszcz (PL); GAWARECKA, Katarzyna, PL-02-665 (PL); CHOJNACKI, Tadeusz, PL-01-318 Warszawa (PL); SKORUPINSKA-TUDEK, Karolina, PL-01-929 Warszawa (PL); KULA-SWIEZEWSKA, Ewa, PL-03-707 Warszawa (PL); RAK, Monika, PL-38-223 Osiek (PL); MADEJA, Zbigniew, PL-30-619 Kraków (PL); MASNYK, Marek, PL-00-432 Warszawa (PL); CHMIELEWSKI, Marek, PL-01-572 Warszawa (PL); OCHALEK, Anna, PL-38-214 Biezdziedza (PL)
(74) Representative: Twardowska-Czerwinska, Aleksandra
(86) International application number: PCT/PL2015/000093
(87) International publication number: WO 2016/032348

(56) References cited:
- US-A1- 2005 250 723
- RAK M ET AL: "Efficient, non-toxic gene delivery by negatively charged polyprenyl-based lipoplexes: application in RNA delivery and the effects on cell physiology", FEBS JOURNAL, vol. 280, no. Suppl. 1, Sp. Iss. SI, July 2013 (2013-07), page 340, XP009185777, & 38TH CONGRESS OF THE FEDERATION-OF-EUROPEAN-BIOCHEMICAL-SOCIETI ES (FEBS); SAINT PETERSBURG, RUSSIA; JULY 06 -11, 2013
- RAK M ET AL: "Study of the lipofecting activity of polyprenyltrimethylammonium iodides (PTAI): efficient transfection of various cell types with negatively charged serum compatible lipoplexes", FEBS JOURNAL, vol. 279, no. Suppl. 1, Sp. Iss. SI, September 2012 (2012-09), page 105, XP009185782, & 22ND INTERNATIONAL-UNION-OF-BIOCHEMISTRY-AND-MO LECULAR-BIOLOGY (IUBMB) CONGRESS/37TH FEDERATION-OF-E; SEVILLE, SPAIN; SEPTEMBER 04 -09, 2012
- Zbigniew Madeja ET AL: "New cationic polyprenyl derivative proposed as a lipofecting agent", Acta biochimica Polonica, 8 December 2007 (2007-12-08), pages 873-876, XP055207630, Poland Retrieved from the Internet: URL:http://www.actabp.pl/pdf/4_2007/873.pd f [retrieved on 2015-08-13]
- SAFATOV A S ET AL: "Effect of intramuscularly injected polyprenols on influenza virus infection in mice", ANTIVIRAL CHEMISTRY & CHEMOTHERAPY, INTERNATIONAL MEDICAL PRESS, GB, vol. 11, no. 3, 1 January 2000 (2000-01-01), pages 239-246, XP009153851, ISSN: 0956-3202
- OLGA GAWRYS ET AL: "Cationic derivative of polyprenol, a potential component of liposomal drug carriers, does not alter renal function in rats", EUROPEAN JOURNAL OF LIPID SCIENCE AND TECHNOLOGY, vol. 116, no. 5, 26 May 2014 (2014-05-26), pages 659-662, XP055207642, ISSN: 1438-7697, DOI: 10.1002/ejlt.201300489

## Description

The presented invention is related to a pharmaceutical composition and use of composition in vaccine manufacturing. More specifically, the presented invention is related to use of carriers based on cationic derivatives of polyprenols used as DNA vaccine carriers, easily transported and stored.

One of the biggest achievements of medicine in disease protection was the discovery and first use of vaccinations. It took place over 200 years ago and exerted enormous influence on the levels of prophylaxy of many diseases throughout the world. Over many years of development of production and application techniques of new vaccine types their influence is not waning, and even on the contrary - it becomes an even more important element of care related to health of societies and individual human beings. It often also gives hope for finding a way to eliminate diseases currently classified as incurable. One of the most important preparations opening a range of possibilities and a perspective of new applications include DNA vaccines, creation and use of which enabled intensive development of tools for delivering nucleic acids into cells. The promising potential of DNA vaccines in prophylaxy and therapy of many diseases stimulates search for efficient and safe carriers of genetic material, which could be used *in vivo.* However, such carriers must meet additional criteria - in addition to those required for effective introduction of genes into cells. Because antigens themselves, introduced into the body, are often not immunogenic enough to achieve a satisfactory level of immunological responses, in addition to DNA introduction, the carriers should also ensure possibility of simultaneous introduction of immunomodulating substances or themselves act as adjuvants in order to enhance response to an antigen.

Some of reagents often used to deliver DNA vaccines include reagents used in lipofection - a method of nucleic acids transfer based on use of cationic lipids, which may spontaneously interact with negatively charged nucleic acids, forming lipoplexes (complexes lipids-nucleic acids). Non-specific interaction with the surface of cell membrane stimulates lipoplex uptake by cells, and presence of other (helper) lipids e.g. DOPE (1,2-dioleyl-sn-glycero-3- phosphatidylethanoloamine) or DC-cholesterol [{3ß-[N-(N',N'-dimethylaminoethane)-carbamoyl]cholesterol} hydrochloride] modifies properties of lipoplexes, facilitating nucleic acids release from endosomes.

The large potential of cationic lipids as DNA vaccine carries, as well as adjuvants is the reason behind intensive studies aimed at development of efficient and safe lipid tools (Korsholm 2012, Christensen 2007). Many trials confirmed the action of lipid-based adjuvants (Carroll 2014, Firouzmand 2013), however, no ideal lipid ingredient intended for use in vaccines has been hitherto developed. This may result from the enormous variety of vaccines and methods of their administration, diseases and conditions, bodily reactions and various side effects. The search for ideal carriers and adjuvants is additionally complicated by the variety of mechanisms involved in the process of effective immunisation, which results in high level of complexity of mutual relationships between vaccine ingredients and safety of their use and efficiency.

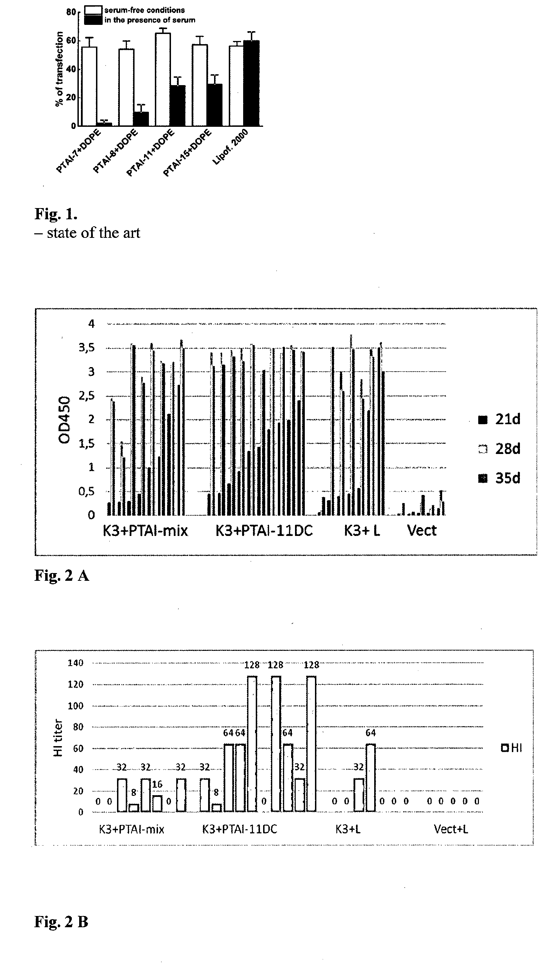

Despite the fact that cationic derivatives of polyprenols with lipofection activity are known in the art (Madeja et al. 2007, patent PL211824), they have not been used as DNA vaccine carriers, nor as adjuvants. The existing solution presented in patent disclosure PL211824 does not indicate a possibility of using polyprenols in serum conditions (in presence of serum), which means that hitherto nothing stimulated specialists in the art to attempt *in vivo* immunisation (Fig. 1 - state of the art). In addition, the described method of mixture preparation does not ensure long-term stability, and in addition it is inconvenient for use in vaccines. It is a serious drawback of solutions known in the art, since vaccines require long-term stability and they should be convenient in storage and transport as well as simple in use.

Document Rak et al.: "Efficient, non-toxic gene delivery by negatively charged polyprenyl-based lipoplexes: application in RNA delivery and the effects on cell physiology" (FEBS Journal, vol. 280, no. Suppl. 1, Sp. Iss. SI, July 2013 (2013-07), page 340, XP009185777) discloses a class of polyprenyl-based cationic lipds for delivery of DNA and RNA molecules.

Document Rak et al.: "Study of the lipofecting activity of polyprenyltrimethylammonium iodides (PTAI): efficient transfection of variouss cell types with negatively charged serum compatible lipoplexes" (FEBS JOURNAL, vol. 279, no. Suppl. 1, Sp. Iss. SI, September 2012 (2012-09), page 105, XP009185782) discloses a new class of polyprenyl-based cationic lipids for delivery of DNA and RNA molecules.

Document Zbigniew Madeja et al.: "New cationic polyprenyl derivative proposed as a lipofecting agent" (Acta biochimica Polonica, 8 December 2007 (200-12-08), pages 873-876, SP055207630) discloses the use of polyprenyl-based cationic lipids for gene delivery into mammalian cells, i.e. as a lipofecting agent.

Document SAFATOV et al.: "Effect of intramuscularly injected polyprenols on influenza virus infection in mice" (ANTIVIRAL CHEMISTRY & CHEMOTHERAPY, INTERNATIONAL MEDICAL PRESS, GB, vol. 11, no. 3, 1 January 2000 (2000-01-01), pages 239-246, XP009153851) discloses that emulsions of polyprenols can be administered to mice via intramuscular route, affording protection from challenge with an H3N2 Influenza Virus A strain.

US 2005/250723 A1 discloses vaccines based on DNA or RNA constructs expressing the antigen of interest.

The aim of the presented invention is to provide new lipid carriers used for introduction of DNA vaccines into eukariotic organisms, said carriers also acting as adjuvants, without the aforementioned limitations. More precisely, the aim of the presented invention is to provide use of compositions containing nucleic acids carriers composed of cationic derivatives of polyprenols PTAI with variable polyprenyl chain length (6-15 isoprene units) of formula (I) and additional (helper) lipids.

Implementation of thus presented aim and solution of problems described in the art, related to development of an invention enabling use of carriers based on cationic derivatives of polyprenols, replying to the known in the art need of introduction of DNA vaccines easy in transport and storage, has been achieved with this invention. Unexpectedly, the change to composition and preparation of compositions containing cationic derivatives of polyprenols led to their effective and convenient use in efficient immunisation, allowing to obtain vaccines with long-term stability, which significantly improved their convenient use without the need of vaccine preparation directly before each application.

The subject of the invention is a pharmaceutical composition comprising a carrier and a nucleic acid encoding influenza virus antigen(s), wherein a nucleic acid as the active substance and nucleic acid carriers comprised of cationic derivatives of polyprenols PTAI with polyprenyl chain length of n, where n denotes the number of isoprene units in a range from 5 to 30, according to formula (I) 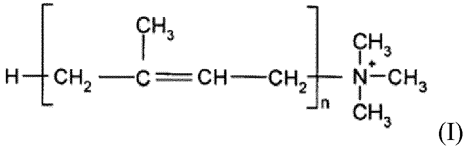 for use in vaccination against influenza virus.

Preferably, the composition comprises cationic derivatives of polyprenols PTAI with the length of polyprenyl chain, wherein n denotes from 6 to 12 isoprene units.

Preferably, the PTAI content is at least 20% (w/w) of the mixture.

Preferably, the composition comprises helper lipids and wherein the helper lipids content is at least 5% (w/w) of the mixture.

Preferably, the helper lipids are lipids used in lipofection.

Preferably, the helper lipids are selected from a group consisting of cholesterol derivatives, phosphatidylcholine derivatives with attached higher fatty acid groups, phosphatidylethanolamine derivatives with attached higher fatty acid groups, phosphatidylserine derivatives.

Preferably, the helper lipids are selected from a group consisting of DOPE (1,2-dioleyl-sn-glycero-3-phosphatidylethanolamine), cholesterol, DC-cholesterol [{3ß-[N-(N',N'-dimethylaminoethane)-carbamoyl]cholesterol}hydrochloride], DOPC: (1,2-dioleyl-sn-glycero-3-phosphatidylcholine), DMPC (1,2-dimiristyl-sn-glycero-3-phosphatidylcholine), DMPE (1,2-dimiristyl-sn-glycero-3-phosphatidylethanolamine), DLPC (1,2-dilauryl-sn-glycero-3-phosphatidylcholine), DPPC (1,2-dipalmityl-*sn*-glycero-3-phosphatidylcholine), phosphatidylcholine, phosphatidylethanolamine, DPPE (1,2-dipalmityl-*sn*-glycero-3-phosphatidylethanolamine), DLPE (2-dilauryl-*sn*-glycero-3-phosphatidylethanolamine), DOPS (1,2-dioleyl-*sn*-glycero-3-phosphatidylserine), POPC (1-palmityl-2-oleyl-*sn-*glycero-3-phosphatidylcholine), PDME (phosphatidyl-N,N-dimethylethanolamine), DSPE (1,2-distearyl-*sn*-glycero-3- phosphatidylethanolamine), DSPC (1,2-distearyl-*sn*-glycero-3-phosphatidylcholine), POPE (1-palmityl-2-oleyl-*sn*-glycero-3-phosphatidylethanolamine) or PMME (phosphatidyl-monomethylethanolamine).

Another subject of the invention is a composition characterized above for manufacturing a DNA vaccine wherein the nucleic acid is a DNA having a sequence of SEQ ID NO: 1.

The subject of the invention is depicted in the figures, where:
**Figure 1** presents Graph A (state of the art) presenting effectiveness of lipofection of DU145 cells using PTAI+DOPE in serum-free conditions and in presence of serum using DMEM F12 HAM medium. Lipoplex concentrations (provided per well of a 24-well plate): PTAI-7+DOPE - 2.5 µg/well, PTAI-8+DOPE -3 µg/well, PTAI-11+DOPE - 4 µg/well, PTAI-15+DOPE - 5 µg/well. These values are provided as average values ± SD. Lipof. 2000 - Lipofectamine 2000 Reagent - reagent used as positive control sample. (Rak 2014).
**Figure 2** presents a comparison of immunological response (levels of anti-HA IgY) of chickens immunised using a DNA preparation (K3 plasmid, 125 µg) mixed with commercial carrier Lipofectin^{®} (K3+L) or cationic derivatives of polyprenols (K3+PTAI-mix and K3+PTAI-11DC). Negative immunisation control was provided by a group of chickens (Vect) vaccinated using a mixture of an empty vector with Lipofectin (2 chickens) or PTAI (3 chickens). The level of specific anti-HA IgY antibodies was determined in blood sera (1:200 dilution) collected on the 21st, 28th and 35th day of life (21d, 28d, 35d) using an ELISA test. Results obtained for single/individual chickens are presented in panel A, while HI levels determined in a hemagglutination inhibition (HI) test for sera collected from individual chickens on the 35th day of life are presented in panel B. The results confirm effective action (better than observed with Lipofectin) of the polyprenol mixtures used, in particular for PTAI-11DC.

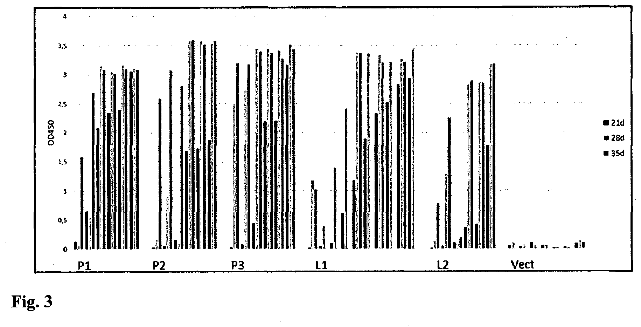

**Figure 3** presents levels of immunological response (anti-HA IgY) in sera (1:200 dilution) of chickens immunised with various DNA mixtures with carriers prepared according to diagram A. Sera collected on the 21st, 28th and 35th day of life were tested using an ELISA test. The presented results indicate highly efficient action of the PTAI carriers used.

### Diagram A. Composition of the vaccine used in the experiment containing a mixture of PTAI composition (PTAI-11+DOPE+DC-cholesterol) with DNA.

Vect - empty pCI vector without the HA coding sequence (negative control)

| Group number | Composition | PTAI-11:DNA (w/w) |
|---|---|---|
| P1 | PTAI+125 µg DNA (K3) | 0.8:1 |
| P2 | PTAI+62 µg DNA (K3) | 1.6:1 |
| P3 | PTAI+62 µg DNA (K3) | 0.8:1 |
| L1 | Lipofectin (20µl)+125 µg DNA (K3) | N/A |
| L2 | Lipofectin (10µl)+62 µg DNA (K3) | N/A |
| Vect | Lipofectin (20µl)+125 µg DNA (pCI) | N/A |

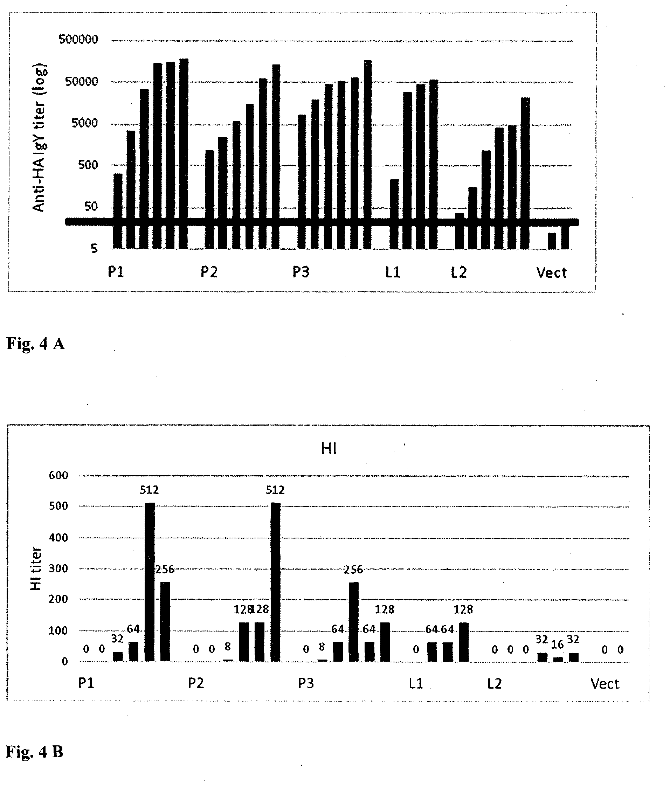

**Figure 4A** presents the anti-HA titers in sera collected from chickens on the 35th day of life determined in the ELISA test using double serial dilutions of the determined sera. The red horizontal line denotes the highest serum titer in control chickens. **Figure 4B** presents HI levels of the tested sera. The results indicate high effectiveness levels of the tested carriers (P1, P2, P3).

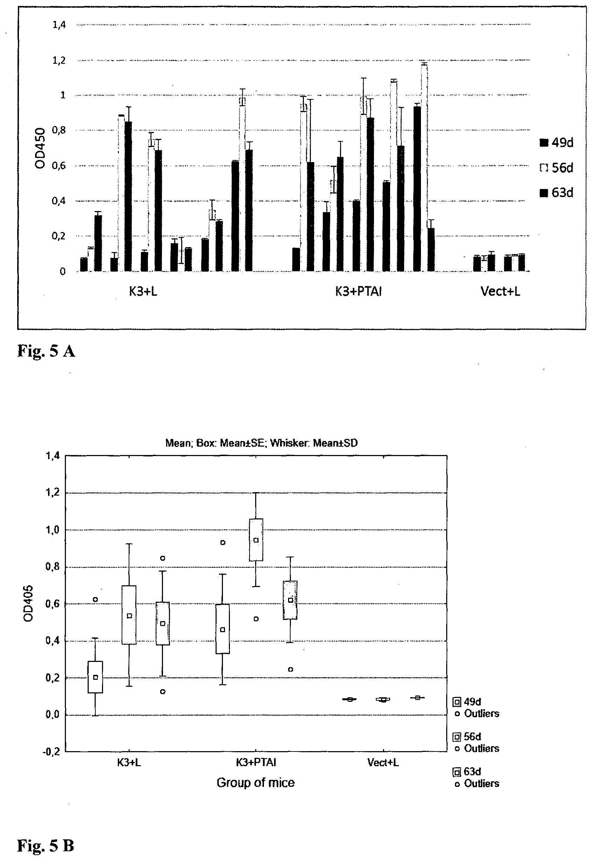

**Figure 5** presents levels of induced immunological response (anti-HA IgG) in mice vaccinated with DNA preparations (K3 plasmid, 10µg) mixed with Lipofectin (K3+L) or with cationic derivatives of polyprenols (K3+PTAI). Results obtained for individual specimens as well as average values for entire groups have been presented in panels A and B, respectively. They indicate higher efficiency of PTAI action when compared to Lipofectin - the higher level of specific antibodies. The negative immunisation control included a group of mice (Vect+L) immunised using a mix of the empty vector and Lipofectin.

Diagram A presents detailed composition of DNA preparations used for immunisation of chickens in the experiment described in Example 2.

In order to provide better understanding of the invention, example solutions according to the invention are presented below.

### Examples

### Example 1. Preparation and use of selected compositions for chicken immunisation against influenza virus and their comparison with a commercially available reagent.

PTAI-6,7,8, PTAI-11 and helper lipids - DOPE, DC-cholesterol were dissolved in 99.8% ethanol, in respective concentrations - 20; 10; 25; 33 mg/ml. They were mixed in appropriate ratios in order to achieve the following molar rations in the mixtures:
PTAI-6,7,8+DOPE - 1,5:1
PTAI-11+DOPE+DC-cholesterol -1:1:1

Then the obtained mixtures were mixed with DMEM F-12 HAM cell culture medium without additions of serum and antibiotics and intensely mixed for 3 minutes - ethanol concentration in thus prepared compositions was as follows:
PTAI-6,7,8+DOPE - 11.4%
PTAI-11+DOPE+DC-cholesterol - 20.7%

The ready compositions were stored at 4°C and used for immunisation within 5 days after preparation.

Preparations for immunisation were prepared by mixing PTAI with DNA (K3 plasmid containing H5 HA coding sequence from H5N1 influenza virus; patent application P396415; SEQ ID No. 1) in order to achieve weight ratio of 0.9:1 for PTAI-6,7,8:DNA and 0.8:1 for PTAI-11:DNA.

DNA coding the influenza antigen was used only as an example. The presented solution can include any other DNA vaccine.

Next the PTAI/DNA mixtures were incubated for 30 min at room temperature and administered to 7-day old broiler chickens as intramuscular injections containing 125 µg DNA. A reminder dose was administered two weeks later (21st day of life). Positive control includes chickens immunised with DNA preparations, wherein PTAI was replaced with commercially available reagent Lipofectin^{®} (Invitrogen, Life Technologies). Blood samples were collected three times, on the 21st, 28th and 35th day of life, namely and respectively, on the day of reminder dose administration as well as one and two weeks later.

Immunisation effectiveness was evaluated in an ELISA test. Plates (96-well) were coated using recombined homologous H5 HA antigen, using an amount of 300 ng/well. Goat anti-chicken IgY antibodies (Fc-specific) conjugated with HRP were used in IgY detection. **Figure 2** presents results indicating high effectiveness levels of the PTAI compositions used in chickens immunisation against influenza virus using a DNA vaccine (subject of the P396415 patent application. Results obtained for PTAI-6,7,8+DOPE are denoted as PTAI-mix, whilst those obtained for PTAI-11+DOPE+DC-cholesterol are denoted as PTAI-11DC. Panel A presents results of the ELISA test obtained for sera (at a 200-fold dilution) collected from individual chickens on the 21st, 28th and 35th day of life, while results of the HI test in sera collected on the 35th day of life are presented in panel B.

### Example 2. Use of PTAI-11+DOPE+DC-cholesterol mixture with various quantities of DNA in immunisation of chickens against influenza virus.

A PTAI composition (PTAI-11+DOPE+DC-cholesterol) prepared according to the description provided in Example 1 was mixed with 125 µg or 62,5 µg of plasmid DNA. Thus, three mixtures with various DNA doses were obtained: (1) P1 (125 µg DNA per vaccine dose), wherein the PTAI-11:DNA weight ratio was 0.8:1, (2) P2, (62,5 µg DNA per vaccine dose), wherein the PTAI-11:DNA weight ratio was 1.6:1 and (3) P3 (62,5 µg DNA per vaccine dose), wherein the PTAI-11:DNA weight ratio was 0.8:1. The prepared vaccine doses were incubated at room temperature for 30 minutes. Vaccine doses containing commercially available transfection reagent Lipofectin instead of PTAI (mixtures L1 and L2) were prepared simultaneously. The negative immunisation control included a group of chickens (Vect) immunised using a mixture of the empty vector and Lipofectin. Composition of mixtures used in vaccinations is provided in Diagram A. Laying chickens were immunised intramuscularly, twice, on the 7th and 21st day of life. Division into groups according to the preparation used in vaccinations is provided on Diagram A. Blood samples were collected from the wing vein on the 21st, 28th and 35th day of life. Immunological response was studied using an ELISA test. Results of the test confirmed high efficiency levels of the PTAI used as a DNA vaccine carrier, **Figure 3** presents ELISA results indicating high level of anty-HA antibodies in sera (200-fold dilution) of the immunised animals. 96-well plate was coated using 300 ng of a recombined HA antigen, purified from a baculovirus system. IgY was detected using goat anti-chicken IgY (Fc specific) antibodies conjugated with HRP.

**Figure 4A** presents levels of anti-H5 HA antibodies in double serial dilutions of sera collected on the 35th day of life. **Figure 4B** presents results of the HI test for the same sera. The level of anti-HA antibodies was determined as a inverse value of the highest serum dilution at which a result higher than the estimated value: background+3xSD (3-times the value of the standard deviation) was obtained. Results are presented using logarithmic scale. If there was no need for dilutions higher than the standard 200-fold dilutions (readout <1 for the 200-fold dilution), the levels were determined by multiplying the OD value read out in the ELISA test by 200, namely by the inverse value of serum dilution used in the ELISA test.

### Example 3. Use of a PTAI-10-14+DOPE+DC-cholesterol mixture with DNA as a vaccine for mice immunisation against influenza virus.

The mixture of PTAI composition [PTAI-10-14 (10, 11, 12, 13, 14)+DOPE+DC-cholesterol] with plasmid DNA (10µg), wherein the PTATDNA weight ratio is 0.8:1 was prepared according to the description in Example 1. Female BALB/c mice were immunised intramuscularly twice, on the 35th and 49th day of life using a K3 mixture with the PTAI composition (K3+PTAI) or K3 with Lipofectin (K3+L). The negative control used included a mixture containing control DNA (the vector without the HA-coding insert) with Lipofectin (Vect+L). Blood samples were collected on the 49th, 56th and 63rd day of life. Presence of anti-HA specific antibodies was determined using ELISA tests. The plate used in tests was coated using 300 ng of the recombined HA H5 antigen. Goat anti-mouse IgG antibodies conjugated with AP were used in IgG detection. **Figure 5** presents the level of anti-HA IgG antibodies in 1:100 diluted sera. Results obtained for sera collected from individual mice have been presened in panel A, average values calculated for respective immunised groups are presented in panel B.

### LITERATURE

Carroll TD, Matzinger SR, Barry PA, McChesney MB, Fairman J, Miller CJ, Efficacy of influenza vaccination of elderly rhesus macaques is dramatically improved by addition of a cationic lipid/DNA adjuvant, J Infect Dis. 2014; 209(1): 24-33.
Chojnacki T, Ciepichal E, Jankowski W, Kula-Świeżewska E, Chmielewski M, Masnyk M, Lysek, Madeja Z, Rak M, Trimetyloaminowe pochodne poli-cis i poli-trans liniowych oligomerów izoprenowych, spos6b ich wytwarzania oraz ich zastosowanie. PL 211824 B1.
Christensen D, Korsholm KS, Rosenkrands I, Lindenstrøm T, Andersen P, Agger EM, Cationic liposomes as vaccine adjuvants, Expert Rev Vaccines. 2007; 6(5): 785-96.
Firouzmand H, Badiee A, Khamesipour A, Heravi Shargh V, Alavizadeh SH, Abbasi A, Jaafari MR, Induction of protection against leishmaniasis in susceptible BALB/c mice using simple DOTAP cationic nanoliposomes containing soluble Leishmania antigen (SLA), Acta Trop. 2013; 128(3): 528-35.
Korsholm KS, Andersen PL, Christensen D, Cationic liposomal vaccine adjuvants in animal challenge models: overview and current clinical status, Expert Rev Vaccines. 2012; 11(5): 561-77.
Madeja Z, Rak M, Wybieralska E, Rózański I, Masnyk M, Chmielewski M, Lysek R, Chojnacki T, Jankowski W, Ciepichal E, Swiezewska E, Tekle M, Dallner G, New cationic polyprenyl derivative proposed as a lipofecting agent, Acta Biochim Pol. 2007 54(4): 873-6.
Rak M, doctoral thesis "Application of cationic polyprenyl derivatives as nucleic acids carriers in lipofection", UJ, 2014
Stachyra A, Góra-Sochacka A, Sawicka R, Florys K, S 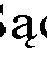 czyńska V, Olszewska M, Pikula A, Śmietanka K, Minta Z, Szewczyk B, Zagórski W, Sirko A. 2014. Highly immunogenic prime-boost DNA vaccination protects chickens against challenge with homologous and heterologous H5N1 virus. - Trials Vaccinol. 3: 40-46; http://dx.doi.org/10.1016/j.trivac.2014.02.002

## Claims

1. A pharmaceutical composition comprising a carrier and a nucleic acid encoding influenza virus antigen(s), wherein a nucleic acid as the active substance and nucleic acid carriers comprised of cationic derivatives of polyprenols PTAI with polyprenyl chain length of n, where n denotes the number of isoprene units in a range from 5 to 30, according to formula (I) 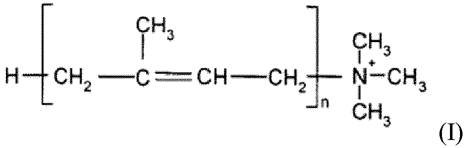 for use in vaccination against influenza virus.

2. Composition for use of Claim 1, **characterised in that** it comprises cationic derivatives of polyprenols PTAI with the length of polyprenyl chain, wherein n denotes from 6 to 12 isoprene units.

3. Composition for use of Claim 1 or 2 **characterised in that** the PTAI content is at least 20% (w/w) of the mixture.

4. Composition for use of according to any of claims 1 to 3, **characterised in that** it comprises helper lipids and wherein the helper lipids content is at least 5% (w/w) of the mixture.

5. Composition for use of according to claim 4, **characterised in that** the helper lipids are lipids used in lipofection.

6. Composition for use of according to claim 5, wherein the helper lipids are selected from a group consisting of cholesterol derivatives, phosphatidylcholine derivatives with attached higher fatty acid groups, phosphatidylethanolamine derivatives with attached higher fatty acid groups and phosphatidylserine derivatives.

7. Composition for use according to claim 6, wherein the helper lipids are selected from a group consisting of DOPE (1,2-dioleyl-sn-glycero-3-phosphatidylethanolamine), cholesterol, DC-cholesterol [{3ß-[N-(N',N'-dimethylaminoethane)-carbamoyl]cholesterol}hydrochloride], DOPC: (1,2-dioleyl-sn-glycero-3-phosphatidylcholine), DMPC (1,2-dimiristyl-sn-glycero-3-phosphatidylcholine), DMPE (1,2-dimiristyl-sn-glycero-3-phosphatidylethanolamine), DLPC (1,2-dilauryl-sn-glycero-3-phosphatidylcholine), DPPC (1,2-dipalmityl-*sn*-glycero-3-phosphatidylcholine), phosphatidylcholine, phosphatidylethanolamine, DPPE (1,2-dipalmityl-*sn*-glycero-3-phosphatidylethanolamine), DLPE (2-dilauryl-*sn*-glycero-3-phosphatidylethanolamine), DOPS (1,2-dioleyl-*sn*-glycero-3-phosphatidylserine), POPC (1-palmityl-2-oleyl-*sn*-glycero-3-phosphatidylcholine), PDME (phosphatidyl-N,N-dimethylethanolamine), DSPE (1,2-distearyl-*sn*-glycero-3-phosphatidylethanolamine), DSPC (1,2-distearyl-*sn*-glycero-3- phosphatidylcholine), POPE (1-palmityl-2-oleyl-*sn*-glycero-3-phosphatidylethanolamine) and PMME (phosphatidyl-monomethylethanolamine).

## Patentansprüche

1. Pharmazeutische Zusammensetzung, umfassend einen Träger und eine Nukleinsäure, die für Influenzavirus-Antigen(e) kodiert, wobei eine Nukleinsäure als Wirkstoff und Nukleinsäureträger kationische Polyprenolderivate von PTAI mit einer Polyprenylkettenlänge von n umfassen, wobei n die Anzahl der Isopren-Einheiten in einem Bereich von 5 bis 30 bezeichnet, gemäß der Formel (I) 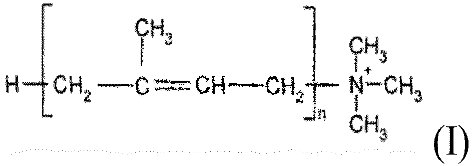 zur Verwendung bei der Impfung gegen das Influenzavirus.

2. Zusammensetzung zur Verwendung nach Anspruch 1, **dadurch gekennzeichnet, dass** sie kationische Derivate von Polyprenolen PTAI mit der Länge der Polyprenylkette umfasst, wobei n 6 bis 12 Isopreneinheiten bezeichnet.

3. Zusammensetzung zur Verwendung nach Anspruch 1 oder 2, **dadurch gekennzeichnet, dass** der PTAI-Gehalt mindestens 20% (w/w) der Mischung beträgt.

4. Zusammensetzung zur Verwendung nach einem der Ansprüche 1 bis 3, **dadurch gekennzeichnet, dass** sie Helferlipide umfasst und dass der Gehalt an Helferlipiden mindestens 5% (w/w) der Mischung beträgt.

5. Zusammensetzung zur Verwendung nach Anspruch 4, **dadurch gekennzeichnet, dass** die Helferlipide Lipide sind, die bei der Lipofektion verwendet werden.

6. Zusammensetzung zur Verwendung nach Anspruch 5, wobei die Helferlipide aus einer Gruppe ausgewählt sind, die aus Cholesterinderivaten, Phosphatidylcholinderivaten mit gebundenen höheren Fettsäuregruppen, Phosphatidylethanolaminderivaten mit gebundenen höheren Fettsäuregruppen und Phosphatidylserinderivaten bestehen.

7. Zusammensetzung zur Verwendung nach Anspruch 6, wobei die Helferlipide ausgewählt sind aus einer Gruppe bestehend aus DOPE (1,2-Dioleyl-sn-Glycero-3-Phosphatidylethanolamin), Cholesterin, DC-Cholesterin [{3ß-[N-(N',N'-Dimethylaminoethan)-Carbamoyl]Cholesterin}Hydrochlorid], DOPC: (1,2-Dioleyl-sn-Glycero-3-Phosphatidylcholin), DMPC (1,2-Dimiristyl-sn-Glycero-3-Phosphatidylcholin), DMPE (1,2-Dimiristyl-sn-Glycero-3-Phosphatidylethanolamin), DLPC (1,2-Dilauryl-sn-Glycero-3-Phosphatidylcholin), DPPC (1,2-Dipalmityl-sn-Glycero-3-Phosphatidylcholin), Phosphatidylcholin, Phosphatidylethanolamin, DPPE (1,2-Dipalmityl-sn-Glycero-3-Phosphatidylethanolamin), DLPE (2-Dilauryl-sn-Glycero-3-Phosphatidylethanolamin), DOPS (1,2-Dioleyl-sn-Glycero-3-Phosphatidylserin), POPC (1-Palmityl-2-Oleyl-sn-Glycero-3-Phosphatidylcholin), PDME (Phosphatidyl-N,N-Dimethylethanolamin), DSPE (1,2-Distearyl-sn-Glycero-3-Phosphatidylethanolamin), DSPC (1,2-Distearyl-sn-Glycero-3-Phosphatidylcholin), POPE (1-Palmityl-2-Oleyl-sn-Glycero-3-Phosphatidylethanolamin) und PMME (Phosphatidyl-Monomethylethanolamin).

## Revendications

1. Composition pharmaceutique composée d'un porteur et d'un acide nucléique codant pour l'antigène du virus de la grippe (s), dans lequel un acide nucléique est la substance active et des porteurs d'acide nucléique sont composés de dérivés cationiques des polyprénols PTAI d'une longueur de chaîne polyprényle de « n », où n désigne le nombre d'unités isopréniques dans une plage comprise entre 5 et 30, selon la formule (I) 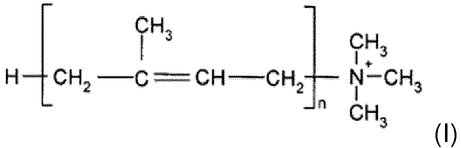 à utiliser dans la vaccination contre le virus de la grippe.

2. Composition à utiliser dans la revendication 1, **caractérisée par le fait qu'**elle comprend des dérivés cationiques des polyprénols PTAI avec la longueur « n » de la chaîne polyprényle, où « n » désigne de 6 à 12 unités d'isoprène.

3. Composition destinée à l'utilisation selon les revendications 1 ou 2, **caractérisée par** une teneur en PTAI d'au moins 20% (m/m) du mélange.

4. Composition destinée à être utilisée conformément à l'une des revendications 1 à 3, **caractérisée par le fait qu'**elle contient des lipides auxiliaires et dont la teneur en lipides auxiliaires est d'au moins 5% (m/m) du mélange.

5. Composition à utiliser selon la revendication 4, **caractérisée par le fait que** les lipides auxiliaires sont des lipides utilisés dans la lipofection.

6. Composition à utiliser selon la revendication 5, dans laquelle les lipides auxiliaires sont sélectionnés parmi un groupe composé de dérivés du cholestérol, de dérivés de la phosphatidylcholine avec des groupes d'acides gras plus élevés, de dérivés de la phosphatidyléthanolamine avec des groupes d'acides gras plus élevés et de dérivés de la phosphatidylsérine.

7. Composition à utiliser selon la revendication 6, dans laquelle les lipides auxiliaires sont sélectionnés parmi un groupe composé de DOPE (1,2-dioléyl-sn-glycérol-3-phosphatidyléthanolamine), de cholestérol, de cholestérol DC [{3β-[N-(N',N'-diméthylaminoéthane)-carbamoyl]cholestérol}hydrochlorure], de DOPC: (1,2-dioléoyl-sn-glycérol-3-phosphatidylcholine), DMPC (1,2-dimyristoyl-sn-glycérol-3-phosphatidylcholine), DMPE (1,2-dimyristoyl-sn-glycérol-3-phosphatidyléthanolamine), DLPC (1,2-dilauroyl-sn-glycérol-3-phosphatidylcholine), DPPC (1,2-dipalmitoyl-sn-glycérol-3-phosphatidylcholine), phosphatidylcholine, phosphatidyléthanolamine, DPPE (1,2-dipalmitoyl-sn-glycérol-3-phosphatidyléthanolamine), DLPE (2-dilauroyl-sn-glycérol-3-phosphatidyléthanolamine), DOPS (1,2-dioléoyl-sn-glycérol-3-phosphatidylsérine), POPC (1-palmitoyl-2-oléoyl-sn-glycérol-3-phosphatidylcholine), PDME (phosphatidyl-N,N-diméthyléthanolamine), DSPE (1,2-distearoyl-sn-glycérol-3-phosphatidyléthanolamine), DSPC (1,2-distearoyl-sn-glycérol-3-phosphatidylcholine), POPE (1-palmitoyl-2-oléoyl-sn-glycérol-3-phosphatidyléthanolamine) et PMME (phosphatidyl-monométhyléthanolamine).
